# FASCICULE DE BREVET EUROPEEN

(11) **EP 2 552 463 B1**
(45) Date de publication et mention de la délivrance du brevet: **16.08.2017**
(21) Numéro de dépôt: 11718428.3
(22) Date de dépôt: 28.03.2011
(51) Int. Cl.: A61K 35/51, A61K 35/28, A61K 35/15, A61K 9/00, A61K 47/42, A61K 38/17, A61P 3/10, A61P 9/10, A61P 3/04, A61P 17/18

(54) **COMPOSITIONS PROANGIOGÉNIQUES, LEUR PROCÉDÉ DE PRÉPARATION ET LEURS UTILISATIONS**
PROANGIOGENE ZUSAMMENSETZUNGEN, VERFAHREN ZU IHRER HERSTELLUNG UND VERWENDUNG
PROANGIOGENIC COMPOSITIONS, METHOD FOR PREPARING THEM, AND USES THEREOF

(30) Priorité: 26.03.2010 FR 1052218
(43) Date de publication de la demande: 06.02.2013
(73) Titulaire: Institut des Vaisseaux et du Sang, 75475 Paris Cedex 10 (FR); Université Paris Diderot - Paris 7, 75205 Paris Cedex 13 (FR)
(72) Inventeur: TOBELEM, Gérard, F-75005 Paris (FR); SILVESTRE, Jean-Sébastien, F-92330 Sceau (FR)
(74) Mandataire: Uchida, Kenji
(86) Numéro de dépôt international: PCT/FR2011/050673
(87) Numéro de publication internationale: WO 2011/117559

(56) Documents cités:
- EP-A1- 0 506 574
- EP-A1- 2 047 859
- WO-A1-02/092108
- WO-A2-2007/012764
- FR-A1- 2 799 465
- FR-A1- 2 799 549
- FR-A1- 2 909 559
- FOUBERT P ET AL: "PSGL-1?mediated activation of EphB4 increases the proangiogenic potential of endothelial progenitor cells", JOURNAL OF CLINICAL INVESTIGATION, AMERICAN SOCIETY FOR CLINICAL INVESTIGATION, US LNKD- DOI:10.1172/JCI28338, vol. 117, no. 6, 1 juin 2007 (2007-06-01), pages 1527-1537, XP002439684, ISSN: 0021-9738
- YANG C M ET AL: "Anti-angiogenic effects and mechanisms of polysaccharides from Antrodia cinnamomea with different molecular weights", JOURNAL OF ETHNOPHARMACOLOGY, ELSEVIER SCIENTIFIC PUBLISHERS LTD, IE, vol. 123, no. 3, 25 juin 2009 (2009-06-25) , pages 407-412, XP026150435, ISSN: 0378-8741, DOI: DOI:10.1016/J.JEP.2009.03.034 [extrait le 2009-03-31]
- MAEKAWA HIROMITSU ET AL: "Ephrin-B2 induces migration of endothelial cells through the phosphatidylinositol-3 kinase pathway and promotes angiogenesis in adult vasculature", 20031101; 20031100, vol. 23, no. 11, 1 novembre 2003 (2003-11-01), pages 2008-2014, XP002385797,

## Description

### Domaine Technique

La présente invention se rapporte à de nouvelles compositions proangiogéniques, à leur procédé de préparation et à leurs utilisations notamment en thérapeutique pour la prévention ou le traitement de toute pathologie responsable de complications du type ischémique.

Les compositions proangiogéniques de l'invention ouvrent la voie à de nouvelles stratégies de thérapie cellulaire.

Dans la description ci-dessous, les références entre crochets [ ] renvoient à la liste des références présentée à la fin du texte.

### Etat de la technique

La mise en évidence de l'existence de précurseurs circulants des cellules endothéliales EPC (Endothelial Progenitor Cells en anglais) et de leur potentiel d'utilisation pour stimuler l'angiogenèse et la réparation vasculaire est une avancée importante dans le domaine de la biologie vasculaire.

Ces cellules peuvent être présentes dans la circulation générale soit spontanément soit en réponse à différents stimuli (cytokines pro-inflammatoires, facteurs de croissance, ischémie, statines, etc). Impliquées dans les mécanismes de réparation vasculaire, ces cellules ouvrent une voie thérapeutique intéressante pour le traitement des maladies cardiovasculaires ischémiques.

Des systèmes proangiogéniques du type marqueur cellulaire/ligand spécifique comprenant un précurseur de cellules endothéliales comportant le marqueur cellulaire Eph, en particulier EphB4 ou EphB1, ont été décrits [1]. Dans de tels systèmes, pour stimuler l'angiogenèse, les précurseurs des cellules endothéliales comportant le marqueur Eph, sont activés par un ligand spécifique appartenant à la famille des éphrines. Les précurseurs de cellules endothéliales peuvent être obtenus à partir de cellules mononucléées provenant de la moelle osseuse, du sang périphérique ou du sang de cordon ombilical. Après différenciation, les cellules mononucléées de la moelle osseuse ou du cordon ombilical fournissent une quantité relativement importante de précurseurs de cellules endothéliales qui expriment ou comportent le marqueur Eph. Il existe, dans le sang périphérique un très faible pourcentage des cellules mononuclées qui sont des EPC circulants. Cependant, l'obtention des précurseurs de cellules endothéliales via plusieurs étapes (présélection et tri par cytométrie, culture *ex vivo*, différenciation et maturation des cellules, expansion) nécessite des manipulations complexes et longues, elle est donc couteuse et son rendement est faible.

Il est, par ailleurs, connu que les facteurs de risques dans les maladies cardiovasculaires peuvent induire des désordres vasculaires et cardiovasculaires à travers le dysfonctionnement endothélial, lequel dysfonctionnement peut entraîner la diminution du nombre et de la fonction des EPC qui interviennent dans la réparation des tissus [2]. Une dysfonction et une quantité d'EPC circulant plus faible que la normale ont été observées chez certains individus ayant des facteurs de risques de maladies cardiovasculaires comme, par exemple, l'hypercholestérolémie, le diabète, l'hypertension [3].

Il existe donc un réel besoin de disposer de nouvelles compositions, pouvant stimuler l'angiogenèse au moins avec la même efficacité que les EPC d'origines diverses voire la même intensité que les facteurs angiogènes comme le VEGF, et que ces nouvelles compositions comprennent des cellules facilement accessibles ne nécessitant pas obligatoirement :
- d'étape de présélection et de tri par cytométrie, de culture *ex vivo,* de différenciation et/ou de maturation
   ou
- d'être en mélange avec d'autres cellules pour stimuler l'angiogenèse.

Il existe aussi un réel besoin de disposer de nouvelles compositions, capables de stimuler la fonction angiogenèse, comprenant des cellules dont le nombre reste stable.

Il existe, en outre, un réel besoin de nouvelles compositions proangiogéniques dont la mise en oeuvre est facile, reproductible et de coût réduit.

### Description de l'invention

La présente invention a précisément pour but de répondre à ces besoins en fournissant une composition proangiogénique susceptible d'être obtenue par un procédé comprenant les étapes suivantes :
(i) on se procure du sang périphérique comprenant des cellules mononucléées, lesdites cellules mononucléées que l'on place dans un milieu adapté, lesdites cellules mononucléées présentant des récepteurs aptes à recevoir l'éphrine-B et choisis dans le groupe consistant en EphA et EphB;
(ii) on met en contact lesdites cellules mononucléées dudit sang périphérique de l'étape (i) avec l'éphrine-B associées à une molécule de stabilisation et/ou de liaison, sans soumettre lesdites cellules mononucléées aux étapes de différenciation et de maturation *ex vivo* de manière à provoquer directement l'activation desdites cellules mononucléées.

Les MNC du sang circulant acquièrent, après activation par l'éphrine-B des propriétés proangiogénique comparables ou supérieures à celles des EPC provenant de la moelle osseuse ou du sang de cordon ombilical.

On entend par « des cellules mononuclées du sang circulant » au sens de la présente invention, des cellules mononucléées qui ont atteint un stade de maturation et sont différenciées en monocytes et lymphocytes.

Le terme « sang périphérique » selon l'invention est équivalent au terme « sang circulant ».

Par rapport aux EPC, les cellules mononucléées sont plus directement et plus facilement accessibles dans la mesure où leur isolement, à partir de sang circulant, ne nécessite pas d'étape de présélection et de tri par cytométrie, de culture *ex vivo,* de différenciation et/ou de maturation. Cela facilite donc la mise en oeuvre des compositions proangiogéniques de l'invention mais aussi peut réduire le coût de fabrication desdites compositions.

Dans le cas d'états pathologiques nécessitant des traitements urgents qui ne peuvent pas être différés dans le temps, la rapidité d'accès aux compositions selon l'invention peut constituer un avantage important.

Les compositions de l'invention évitent les problèmes liés à la transfusion de cellules sanguines hétérologues et constituent une voie prometteuse. En effet, les compositions selon l'invention sont adaptées pour une utilisation en autotransfusion.

Jusqu'à présent, il n'avait jamais été imaginé que l'on puisse activer directement les cellules mononucléées (MNC) du sang circulant pour stimuler l'activité proangiogénique. Et d'ailleurs, l'art antérieur n'enseigne que des compositions proangiogeniques à partir de précurseurs de cellules endothéliales lesquelles résultent de différenciation des cellules mononuclées de la moelle osseuse.

Dans les compositions selon l'invention, les cellules mononucléées (MNC) peuvent comporter des récepteurs aptes à recevoir les ligands éphrine-B pour provoquer l'activation desdites cellules.

Les compositions selon l'invention peuvent être sous la forme d'un mélange brut de cellules et de molécules, de diverses natures et d'origines et dont les propriétés peuvent changer ou le nombre peut augmenter, afin de disposer des (MNC) en quantité suffisante pour l'utilisation à laquelle ladite composition est destinée. La quantité des cellules mononucléées présentes dans la composition et/ou dans le lot (ou mélange) de cellules à utiliser, peut être comprise par exemple entre 10⁶ et 10¹².

Les compositions selon l'invention peuvent également être sous une forme « moins brute», débarrassée de certaines cellules et/ou de molécules par des méthodes de purification connues de l'homme du métier. Ces méthodes ne doivent pas détériorer les cellules (MNC). La composition « moins brute», contient, en général, une concentration plus élevée de cellules (MNC) aptes à être stimulées par rapport à la concentration en cellules (MNC) du mélange brut. La quantité des cellules présentes dans la composition et/ou dans le lot (ou mélange) de cellules à utiliser, peut alors être comprise par exemple entre 10⁶ et 10¹¹ de cellules mononucléées.

La composition selon l'invention peut comprendre en outre le sang de cordon ombilical ou la moelle osseuse, ou d'un mélange des deux.

Plus particulièrement, les cellules mononucléées (MNC) du sang circulant peuvent provenir du sang périphérique d'un mammifère

Le terme « mammifère » désigne tout mammifère. A titre d'exemple, on peut citer les animaux domestiques comme les chiens et les chats ; les animaux de ferme comme les porcs, les bovins, les moutons et les chèvres ; les animaux comme les souris et les rats ; les primates comme les singes, les chimpanzés ; et l'homme.

Dans le cadre de la présente invention, « stimuler » l'activité proangiogénique, signifie entraîner l'accroissement et l'amélioration des propriétés proangiogéniques *in vitro* et *in vivo.*

Toujours dans le cadre de l'invention, le terme provoquer « l'activation » des cellules mononucléées (MNC) du sang circulant, désigne provoquer le déclenchement d'une réponse cellulaire de la part des cellules (MNC) différenciées.

Comme déjà indiqué, les compositions selon l'invention comprennent des cellules mononucléées (MNC) du sang circulant comportant des récepteurs aptes à recevoir les ligands L pour provoquer l'activation desdites cellules. Même en présence d'un excès, les ligands L ne se lieront qu'aux récepteurs aptes à les recevoir et non à tous les récepteurs qui peuvent être présents sur les cellules mononucléées (MNC) du sang circulant.

Les cellules mononucléées (MNC) du sang circulant comportent donc des récepteurs aptes à être stimulés par des ligands membranaires non circulants, comme les éphrines.

Ces récepteurs peuvent être par exemple des récepteurs transmembranaires à activité tyrosine kinase, choisis dans le groupe comprenant les EphA, les Eph B.

Les ligands L peuvent être des ligands de la famille des éphrines. Les éphrines peuvent être divisées en deux sous-familles, les ligands de type A étant simplement liés aux membranes plasmiques par un glycosylphosphatidilinositol (GPI) et les ligands B étant transmembranaires avec un domaine intracytoplasmique.

Dans un mode de réalisation de l'invention, le ligand L est une molécule à activité éphrine-B, plus particulièrement une molécule à activité éphrine-B2. Le ligand L peut être l'éphrine-B2.

Les ligands L peuvent être éventuellement associés à une molécule de stabilisation et/ou de liaison. Dans le cadre de la présente invention, le terme « associé » couvre tout type de liaison d'intensité et de propriété différente : à savoir, covalente, ionique, polaire, de Van der Waals et hydrophobe. Les molécules de stabilisation et/ou de liaison peuvent stabiliser et/ou protéger le ligand. Elles peuvent fixer le ligand mais ne peuvent pas se fixer sur les récepteurs.

Dans les compositions selon l'invention, la molécule de stabilisation et/ou de liaison peut être, par exemple, un fragment Fc d'une immunoglobuline. Le fragment Fc, qui résulte de la fragmentation des liaisons peptidiques d'une molécule d'immonuglobuline, ne possède pas de site de fixation des antigènes. Il ne possède donc pas d'activité anticorps. Pour le fragment Fc (ou domaine Fc), il s'agit de la partie de la molécule d'immunoglobuline formée par les régions constantes des 2 chaînes lourdes. C'est ce domaine Fc qui donne ses propriétés à l'immunoglobuline. Le fragment Fc peut être obtenu à partir des immunoglobulines, par exemple, par une action protéolytique de la papaïne, de la pepsine ou toute autre enzyme adaptée.

Un autre objet de l'invention est un matériau proangiogénique comprenant des cellules mononucléées (MNC) du sang circulant activées par des ligands L pour stimuler l'angiogénèse, lesdits ligands L étant éventuellement associés à une molécule de stabilisation et/ou de liaison.

Dans un mode de réalisation de l'invention, le matériau proangiogénique comprend des cellules mononucléées (MNC) du sang circulant stimulées par des molécules d'éphrine-B2 comme ligands, lesdits ligands L étant éventuellement associés à une molécule de stabilisation et/ou de liaison. Dans ce mode de réalisation, lesdites cellules mononucléées (MNC) du sang circulant comportent des récepteurs choisis parmi les Eph A et les EphB, aptes à être stimulés par des molécules d'éphrine-B2.

L'invention concerne également le procédé de préparation d'une composition proangiogénique selon l'invention, comprenant les étapes suivantes :
(i) on se procure des cellules mononucléées (MNC) du sang périphérique que l'on place dans un milieu adapté ;
(ii) on met en contact les cellules mononucléées de l'étape (i), avec les ligands L.

Lesdits ligands étant éventuellement associés à une molécule de stabilisation et/ou de liaison.

Le terme « sang périphérique » selon l'invention est équivalent au terme « sang circulant ». Les cellules mononucléées proviennent du sang périphérique (PBMNC).

En outre, le sang de cordon ombilical ou la moelle osseuse, ou d'un mélange des deux peut être ajouté(e) au sang périphérique comprenant les cellules mononucléées de l'étape.

Les MNC peuvent être isolées à partir du sang périphérique par toute méthode adaptée connue de l'homme du métier, comme par exemple la centrifugation différentielle, le tri par la cytométrie de flux.

Le milieu adapté de l'étape (i) peut être un liquide physiologique comprenant principalement de l'eau. Le milieu adapté peut comprendre au moins 80%, avantageusement au moins 85% et encore plus avantageusement au moins 90% d'eau.

Outre l'eau, le milieu adapté peut comprendre un ou plusieurs éléments choisis parmi :
- les solutés minéraux comme des oligo-éléments et des ions notamment Na⁺, Cl⁻, K⁺, PO₄³⁻, Ca²⁺, SO₄²⁻ ;
- les solutés organiques comme des lipides, des glucides, des acides aminés, de l'urée, de l'acide urique, de la bilirubine, des hormones ;
- les protéines plasmatiques comme l'albumine, des immunoglobulines, du fibrinogène, des lipoprotéines etc.

Le milieu adapté est plus particulièrement un plasma.

Dans certains cas, le plasma peut être du plasma autologue.

Les cellules mononucléées (MNC) du sang circulant sont mises en contact avec des ligands de la famille des éphrines.

Le ligand L est avantageusement une molécule à activité éphrine-B, plus avantageusement une molécule à activité éphrine-B2. Le ligand L dans l'étape (ii) peut être l'éphrine-B2.

Le ligand L peut éventuellement être associé à une molécule de stabilisation/liaison telle que décrite précédemment.

Les cellules mononucléées (MNC) utilisées comportent des récepteurs aptes à être stimulés par des ligands de la famille des éphrines. Ces récepteurs peuvent être par exemple des récepteurs transmembranaires à activité tyrosine kinase, choisis dans le groupe comprenant les EphA, les Eph B.

Le ligand L peut éventuellement être associé à une molécule de stabilisation/liaison telle que décrite précédemment. La molécule de stabilisation/liaison est avantageusement un fragment Fc d'une immunoglobuline.

Dans l'étape (ii), la durée de mise en contact entre les cellules mononucléées (MNC) du sang circulant et les ligands L peut être comprise entre 5 minutes et 6 heures, par exemple entre 20 minutes et 4 heures, par exemple entre 30 minutes et 1 heure.

La mise en contact de l'étape (ii) peut s'effectuer à une température allant de 20 à 50°C, par exemple entre 25 et 40°C, par exemple 37°C.

La mise en contact de l'étape (ii) peut se faire dans une atmosphère favorisant la liaison des ligands L aux récepteurs des cellules (MNC) du sang circulant, par exemple avec des pourcentages en CO₂ et en oxygène variant respectivement entre 2% et 10%, et entre 98% et 90%, la somme des pourcentages de CO₂ et d'oxygène étant égale dans tous les cas à 100%. Plus particulièrement, l'atmosphère choisie est composée de 5% de CO₂ et de 95% d'oxygène.

Dans le procédé de l'invention, on peut, en outre, effectuer une étape de lavage après l'étape (ii). Le lavage peut être effectué avec un milieu adapté tel que décrit précédemment.

L'invention s'étend également aux compositions proangiogéniques susceptibles d'être obtenues selon le procédé de l'invention, ladite composition comprenant des cellules mononucléées (MNC) du sang circulant et des ligands L aptes à activer lesdites cellules mononucléées (MNC) du sang circulant pour stimuler l'activité proangiogénique ;
lesdits ligands L étant éventuellement associés à une molécule de stabilisation et/ou de liaison.

Les compositions proangiogéniques selon l'invention, peuvent être utilisées directement ou sous forme d'une composition pharmaceutique, pour une utilisation comme médicament.

Le matériau proangiogénique selon l'invention, peut également être utilisé directement ou sous forme d'une composition pharmaceutique le contenant, pour une utilisation comme médicament.

Ainsi, l'un des aspects de l'invention peut être la composition proangiogénique ou le matériau proangiogénique, selon l'invention, pour son utilisation comme médicament, en particulier pour le traitement et la régénération des tissus vasculaires qui ont été endommagés notamment au niveau cardiaque, cérébral ou encore périphérique ; ou encore en thérapeutique, notamment pour la prévention ou le traitement de toute pathologie donnant des complications ischémiques comme le diabète, la neuropathie diabétique, l'athérosclérose, l'infarctus de myocarde, les accidents vasculaires cérébraux, l'artériopathie des membres inférieurs, le vieillissement, l'hyperlipidémie, l'hypercholestérolémie, l'obésité, l'hypertension.

Selon un autre de ses aspects, la présente invention concerne des compositions pharmaceutiques comprenant une composition proangiogénique ou un matériau proangiogénique, selon l'invention, et éventuellement un ou plusieurs excipient(s) pharmaceutiquement acceptable(s).

Les compositions pharmaceutiques selon l'invention peuvent être préparées par des procédés connus.

Les excipients sont habituellement des éléments sans activité thérapeutique qui entrent dans la composition d'un médicament ou qui sont utilisés pour sa fabrication. L'excipient a pour fonction d'améliorer l'aspect, d'assurer la conservation, de faciliter la mise en forme et l'administration du médicament. Il sert aussi à acheminer le principe actif vers son site d'action et à contrôler son absorption par l'organisme. Selon la forme pharmaceutique et le mode d'administration souhaités, différents excipients peuvent être utilisés. L'homme du métier est en mesure de choisir les excipients appropriés selon la forme pharmaceutique et le mode d'administration désirés [6].

La quantité de composition proangiogénique ou de matériau proangiogénique directement administrée ou comprise dans les compositions pharmaceutiques est une quantité thérapeutiquement effective. Par quantité effective ou quantité thérapeutiquement effective, on entend une quantité de composition proangiogénique, de matériau proangiogénique, ou de composition pharmaceutique qui conduit à l'effet prophylactique ou thérapeutique désiré. Ladite quantité effective peut être déterminée de manière expérimentale par des procédures pharmaceutiques standard.

Afin d'obtenir l'effet thérapeutique désiré, la dose de principe actif (par exemple l'éphrine B2-Fc) nécessaire pour activer les cellules peut varier entre 10 et 200 microgrammes par ml et le nombre de cellules activées dans le lot à utiliser est de 10⁸ à 10¹¹ cellules par traitement. Bien que ces dosages soient des exemples de situation moyenne, il peut y avoir des cas particuliers où des dosages plus élevés ou plus faibles soient appropriés. De tels dosages font partie de l'invention. Selon la pratique habituelle, le dosage approprié à chaque patient est déterminé par le médecin selon le mode d'administration, le poids et la réponse dudit patient.

Le mode d'administration peut être avantageusement parentérale. L'administration parentérale peut comprendre par exemple l'administration intraveineuse, intramusculaire, sous-cutanée, intrapéritonéale, intra-artérielle, intra-articulaire, intra-cisternale, intradermique, intralésionelle, intraoculaire, intrapleurale, intrathécale, intra-utérine, et intraventriculaire, L'indication thérapeutique à traiter ainsi que les propriétés physicochimiques et biologiques de la composition permet de déterminer la voie d'administration et la formulation les plus appropriées. Différentes formulations et systèmes d'administration sont décrits dans l'état de la technique [5].

Les compositions proangiogéniques ou les matériaux proangiogéniques, de l'invention, peuvent être utilisées pour la fabrication d'un médicament destiné au traitement et à la régénération des tissus vasculaires qui ont été endommagés notamment au niveau cardiaque, cérébral ou encore périphérique. Ils peuvent aussi être utilisées pour la fabrication d'un médicament destiné à la prévention ou au traitement de toute pathologie donnant des complications ischémiques comme le diabète, la neuropathie diabétique, l'athérosclérose, l'infarctus du myocarde, les accidents vasculaires cérébraux, l'artériopathie des membres inférieurs, le vieillissement, l'hyperlipidémie, l'hypercholestérolémie, l'obésité, l'hypertension.

Selon un de ses aspects, la présente invention concerne une composition proangiogénique ou un matériau proangiogénique, selon l'invention, pour le traitement et la régénération des tissus vasculaires qui ont été endommagés notamment au niveau cardiaque, cérébral ou encore périphérique.

Selon un autre de ses aspects, la présente invention concerne une composition proangiogénique ou un matériau proangiogénique, selon l'invention, pour la prévention ou le traitement de toute pathologie donnant des complications ischémiques comme le diabète, la neuropathie diabétique, l'athérosclérose, l'infarctus du myocarde, les accidents vasculaires cérébraux, l'artériopathie des membres inférieurs, le vieillissement, l'hyperlipidémie, l'hypercholestérolémie, l'obésité, l'hypertension.

L'invention est, en outre, relative à l'utilisation d'une molécule à activité éphrine-B2 comme ligand apte à se lier aux récepteurs des cellules mononucléées (MNC) pour déclencher leur activation.

Les compositions proangiogéniques de l'invention peuvent être utilisées en thérapie cellulaire chez le mammifère et en particulier chez l'homme.

La présente invention, selon un autre de ses aspects, concerne également une méthode de prévention ou de traitement des pathologies ci-dessus indiquées qui comprend l'administration d'une composition proangiogénique ou d'une composition pharmaceutique la comprenant, d'un matériau proangiogénique ou d'une composition pharmaceutique le comprenant.

Une première méthode peut consister à :
- isoler les cellules mononucléées (MNC) à partir du sang circulant et/ou de la moelle osseuse ;
- activer les (MNC) *in vitro* par la liaison des ligands L à leurs récepteurs spécifiques présents sur les (MNC), pour améliorer leur propriété proangiogénique ; et
- introduire les cellules (MNC) ainsi activées dans le sang ou directement dans les tissus ischémiques.
Cette méthode permet d'augmenter de manière significative le nombre de cellules (MNC) activées. L'activation *in vitro* des (MNC) peut être, en outre, utile pour ensemencer des prothèses vasculaires par exemple.

Une deuxième méthode peut consister à :
- utiliser directement un échantillon de sang et/ou de moelle osseuse dans son intégralité, contenant des cellules (MNC) éventuellement avec d'autres cellules et des cytokines ;
- ajouter des ligands L audit échantillon ;
- introduire directement le tout sans fractionnement cellulaire.

Chez l'homme, cette méthode peut être qualifiée d'autologue et permet de procéder à des autotransfusions, ce qui permet d'éliminer par exemple tous les problèmes de compatibilité immunologique et de sécurité virale.

### Brève description des figures

- La figure 1 représente les résultats de perfusion cutanée des pattes ischémique et non ischémique de la souris, évalués par vélocimétrie laser-Doppler à imagerie de perfusion (MLDI 5078, commercialisé par la société Moon Instruments).
- La figure 2 représente les résultats de score angiographique des pattes ischémique et non ischémique de la souris, évalués par microangiographie de haute définition (2200, commercialisé par Kodak Dental Systems, France).
- La figure 3 représente les résultats de densité capillaire dans les muscles des pattes ischémique et non ischémique de la souris, évalués par immuno-histomorphométrie quantitative (microscope à fluorescence Leitz et logiciel Histolab commercialisé par Microvision (Evry)).

### EXEMPLES

### Méthodes et Matériels

Les expériences sont effectuées conformément à la législation protégeant les animaux dans le cadre d'expérimentation animale et toute autre loi ou régulation en vigueur en France et en accord avec les Directives de la Communauté Européenne No. 07430 concernant le soin et l'utilisation des animaux de laboratoire). Les protocoles d'étude sont approuvés par le Service de la Protection et la Santé des Animaux et de la Protection de l'Environnement, Ministère de l'agriculture, France.

### Exemple 1 : Préparation d'une composition proangiogénique selon l'invention

### Isolement des MNC

30 à 40 ml de sang périphérique humain ont été fraîchement recueillis dans des tubes revêtus d'héparine (Vacuette 455051, commercialisé par la société Greiner Bio-One GmbH). Les cellules mononucléées (MNC) ont été isolées du sang périphérique par centrifugation de gradient de densité au moyen de Ficoll. Deux heures avant l'injection intraveineuse (iv) des cellules (MNC) dans les animaux, les échantillons de sang ont été dilués 2 fois avec du tampon phosphate salin ou PBS venant de l'anglais phosphate buffered saline (14040 commercialisé par la société Gibco). 30 à 35 ml de l'échantillon dilué du sang ont été placés en couche sur 10ml d'une bande de Ficoll préalablement préparée (1.077g/ml ; P04-60500 commercialisé par la société Pan Biotech). La centrifugation a été réalisée à température ambiante (environ 20°C) pendant 30 minutes à 700 x g (ou 700 fois l'accélération de la pesanteur. La couche blanche des cellules (MNC) a été enlevée de l'interface plasma-Ficoll. Après 2 lavages avec 50 ml de M199 (31153 commercialisé par la société Gibco), les cellules mononucléées ont été comptées et remises en suspension dans un milieu M199 contenant 2% de sérum bovin foetal ou FBS venant de l'anglais Fetal Bovine Serum à une concentration de 3x10⁶ cellules/ml.

### Stimulation des (MNC)

Pour la stimulation, 3x10⁶ cellules mononucléées (MNC) dans 1 ml de milieu M199 contenant 2% de sérum bovin foetal ou FBS ont été incubées avec 15 µg éphrine-B2 (496-EB, R&D Systems) à 37°C pendant 30 minutes sous une atmosphère contenant 5% de CO₂ et 95% d'oxygène. Les cellules (MNC) stimulées ont ensuite été lavées 2 fois avec 10 ml de milieu M199 contenant 2% de FBS afin d'éliminer de l'éphrin-B2 libre.

### Injection des (MNC)

Les (MNC) lavées ont été remises en suspension à une concentration de 10³, 10⁴, et 10⁵ cellules par 100 µl de PBS et ensuite injectées dans la veine de la queue des souris opérées, 5 heures après la ligature de l'artère fémorale dans des conditions stériles.

### Exemple 2 : Activité proangiogénique des compositions selon l'invention

### Préparation des souris nudes athymiques

Des souris adultes nudes athymiques males (6 à 7 semaines d'âge, Harlan) ont été hébergées avec libre accès à la nourriture et à l'eau, au moins 7 jours avant la ligature de l'artère fémorale de droite. Les souris ont été maintenues sur un cycle inversé de nuit/jour de 12h : les souris sont maintenues dans l'obscurité de 7h à 19h puis à la lumière de 19h à 7h, à température ambiante (22°C). Toutes les manipulations sont réalisées dans des conditions exemptes de germes de pathogènes. Les souris ont été gardées dans des cages jetables en plastique comportant des filtres dans leur partie supérieure. La cage, la partie supérieure en filtre, la bouteille d'eau, le plateau de nourriture (Innovive) et la nourriture chow (Harlan) ont été préalablement irradiés aux UV. L'eau potable a été, préalablement à son utilisation, traitée à l'autoclave.

### Ligaturé de l'artère fémorale droite

Une intervention chirurgicale a été réalisée afin de créer une ischémie unilatérale de la patte postérieure dans des conditions stériles. Les souris ont été anesthésiées par inhalation d'isofurane (1,5 à 2,0%). Les réflexes de retrait de patte et de clignement d'oeil ont été complètement supprimés avant l'intervention. L'exposition de l'artère fémorale a été obtenue en réalisant une incision dans la partie centrale de la peau sus-jacente de la patte droite. En utilisant un microscope, l'artère fémorale a été disséquée et séparée de la veine, juste au-dessus du point de départ de la *circomflexa femoris lateratis.* La peau sus-jacente a été ensuite fermée au moyen d'une agrafeuse chirurgicale.

### Evaluation de la perfusion cutanée de fa_patte

L'évaluation du flux sanguin cutanée de la patte par des cellules (MNC) stimulées par éphrine-B2 permet d'évaluer la capacité des cellules (MNC) stimulées à améliorer la perfusion du membre ischémié.

Au 14^{ème} jour de post-ligature, les animaux ont été anesthésiés par injection intra-péritonéale (ip) de pentobarbital (10 mg/kg). Les souris ont été ensuite placées sur une couverture chauffante et la température rectale a été maintenue entre 36,5 et 37°C afin de minimiser l'influence de la température du corps sur la perfusion cutanée de la patte. L'évaluation a été ensuite réalisée au moyen d'un Doppler Laser à imagerie de perfusion (MLDI 5078, commercialisé par la société Moon Instruments). Les résultats de perfusion cutanée pour chaque animal ont été exprimés en rapport de la patte droite ischémiée versus la patte gauche non ischémiée.

### Détermination de la densité artérielle

La détermination de la densité vasculaire par des cellules (MNC) stimulées par éphrine-B2 permet d'évaluer la capacité desdites cellules stimulées à induire le remodelage du réseau vasculaire pré-existant (remodelage vasculaire).

A la suite de l'évaluation de la perfusion cutanée de la patte, la densité vasculaire a été déterminée par de la microangiographie haute définition (2200, commercialisé par Kodak Dental Systems, France). Une laparotomie longitudinale a été réalisée et un cathéter en polyéthylène (PE-10) a été inséré dans l'aorte abdominale à travers laquelle environ 150 µl de produit de contraste (1,67g/ml de sulfate de barium salin) (Fluka-11845, commercialisé par la société Sigma, France), a été injecté. La microangiographie de la patte arrière a ensuite été établie et les images (2 à 3 images par animal) ont été acquises par un transducteur digital aux rayons X. La densité artérielle de chaque patte arrière a été estimée en un pourcentage de pixels par image dans la région de quantification occupée par des artères utilisant par analyse quantitative classique d'images. Les résultats de la densité vasculaire pour chaque animal ont été exprimés en rapport de la patte droite (ischémiée) versus la patte gauche (non ischémiée).

Pour l'analyse immuno-histochimique, les muscles jumeaux du triceps de tous les animaux ont été recueillis inclus dans une matrice Tissue-Tek O.C.T. (commercialisé par la société Finetek Europe) et rapidement congelés au moyen de l'isopentyl prérefroidi dans de l'azote liquide.

### Quantification de la densité capillaire

La quantification de la densité capillaire par des cellules (MNC) stimulées par éphrine-B2 permet d'évaluer la capacité desdites cellules stimulées à induire le développement du système vasculaire à partir d'un réseau capillaire (vasculogenèse et/ou angiogenèse).

Pour l'évaluation de la densité capillaire, chaque échantillon de muscle congelé est sectionné au moyen d'un cryostat (commercialisé par la société Leica). Les sections ont une épaisseur de 7 µm. Le sectionnement a été réalisé à 3 niveaux différents de l'échantillon, séparés par une distance d'environ 300 µm. Les sections ont été incubées pendant 1 heure à température ambiante (20°C), avec de l'isocyanate de fluorescéine ou FITC (fluorescein isocyanate en anglais) conjugué à l'isolectine B4 (100 µl par lame, 10 µg/ml ; L2895 commercialisé par la société Sigma).

Au moyen d'un microscope à fluorescence équipé de filtres appropriés (Observer ZI, commercialisé par la société Zeiss), 6 images (2 images par niveau x 3 niveaux) ont été acquises pour chaque échantillon de muscle ischémique ou non ischémique. Le logiciel HistoLab (Version 7.0, commercialisé par la société Microvision Instrument a été utilisé afin de quantifier la densité capillaire. Les résultats pour chaque animal ont été exprimés en un rapport de densité capillaire dans la patte arrière ischémiée versus la patte arrière non ischémiée.

### Analyses statistiques

Les résultats de la perfusion cutanée de la patte, de la densité artérielle et de la densité capillaire sont exprimés comme une moyenne de pourcentage du groupe témoin traité par PBS (phosphate buffer saline en anglais) ± SEM (erreur sur la moyenne). Les comparaisons entre les différents groupes expérimentaux ont été réalisées au moyen d'une analyse de variance unidirectionnelle (1-way ANOVA en anglais) suivie d'une analyse complémentaire par la méthode PLSD de Fischer (Fischer's Protected Least Significant Différence Method). Les valeurs de P inférieures au seuil de signification 0,05 (P<0,05), ont été considérées significatives.

### Effets de cellules (MNC) du sang circulant stimulées par éphrine-B2 (EFNB2) sur la revascularisation post-ischémique

### Perfusion cutanée de la patte

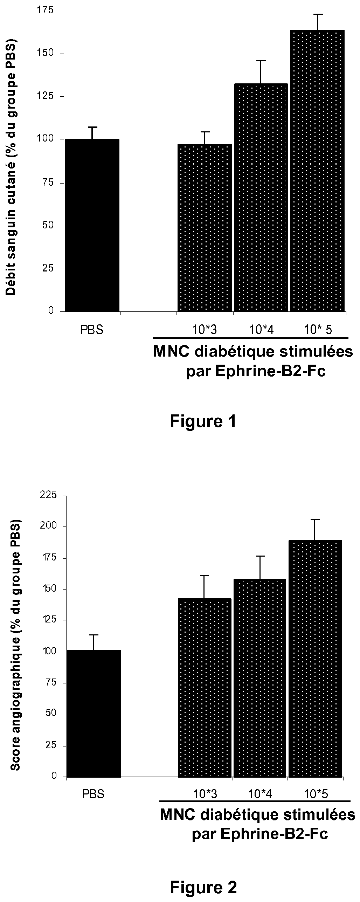

Les cellules (MNC) provenant du sang veineux de patients diabétiques stimulées par EFNB2 à des concentrations de cellules 10³, 10⁴ et 10⁵, ont modifié significativement la perfusion cutanée des pattes en comparaison avec PBS au 14^{ème} jour post-ischémie (97±7%, 132±14% et 163±10% du groupe PBS pour des MNC stimulées par EFNB2 à 10³, 10⁴ et 10⁵ cellules respectivement, figure 1).

### Résultat angiographique

Le score angiographique est significativement augmenté par les MNC provenant de patients diabétiques stimulées avec EFNB2 à des concentrations de cellules de 10³, 10⁴ et 10⁵ comparé à PBS au 14^{ème} jour post-ischémie. (142±19%, 157±19% et 189±17% du groupe PBS pour des MNC stimulées par EFNB2 à 10³, 10⁴ et 10⁵ cellules respectivement, figure 2).

### Densité capillaire

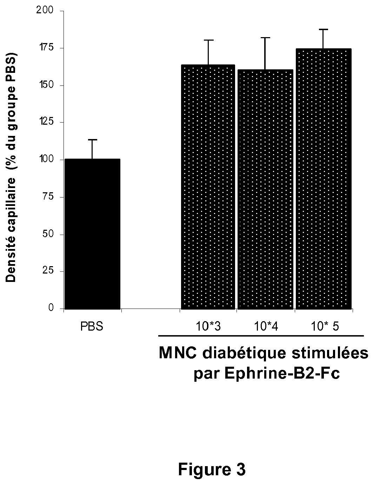

Au 14^{ème} jour post-ligature, la délivrance intraveineuse des cellules MNC stimulées par EFNB2 à des concentrations de cellules 10³, 10⁴ et 10⁵, a significativement augmenté la densité capillaire (163±14%, 156±11% et 171±23% du groupe PBS pour des MNC stimulées par EFNB2 à 10³, 10⁴ et 10⁵ cellules respectivement, figure 3).

En conclusion, les cellules mononucléées du sang périphérique de patient diabétique, activées par ephrinB2-Fc, ont un effet proangiogénique bénéfique très marqué du point de vue fonctionnel (mesure du flux sanguin cutané) et du point de vue structurel (mesures de microangiographies artérielles et de densités capillaires).

### Listes des références

[1] WO 2007/012764.
[2] Quyyumi et al., Can. J. Cardio. 20, 44B-8B, 2004 ; Vasa et al., Circ. Res. 89, E1-7, 2001.
[3] Verma et al., Circulation, 105, 546-549, 2002 ; Vasa et al. Circ. Res. 89, c1-c7, 2001 ; Tepper et al., Circulation, 106, 2781-2786, 2002.
[4] Iba et al., Circulation, 2019-2025, 2002.
[5] Gennaro ed. (2000) Remington's Pharmaceutical Sciences ; Hardman, Limbird et Gilman, eds. (2001) The Pharmacological Basis of Therapeutics.
[6] Pharmacopées USP, JP, EP ; A. Le Hir, J-C. Chaumeil, D. Brossard, Pharmacie galénique, 9éme édition, p.36-93, 2009.

## Revendications

1. Composition proangiogénique susceptible d'être obtenue par un procédé comprenant les étapes suivantes :
(i) on se procure du sang périphérique comprenant des cellules mononucléées, lesdites cellules mononucléées que l'on place dans un milieu adapté, lesdites cellules mononucléées présentant des récepteurs aptes à recevoir l'éphrine-B et choisis dans le groupe consistant en EphA et EphB;
(ii) on met en contact lesdites cellules mononucléées dudit sang périphérique de l'étape (i) avec l'éphrine-B associées à une molécule de stabilisation et/ou de liaison, sans soumettre lesdites cellules mononucléées aux étapes de différenciation et de maturation *ex vivo* de manière à provoquer directement l'activation desdites cellules mononucléées.

2. Composition selon la revendication 1, dans laquelle lesdites cellules mononucléées sont isolées à partir dudit sang périphérique par la centrifugation différentielle.

3. Composition selon la revendication 1 ou 2, dans laquelle le sang de cordon ombilical ou la moelle osseuse, ou d'un mélange des deux sont ajouté au sang périphérique comprenant les cellules mononucléées de l'étape (i).

4. Composition selon l'une quelconque des revendications 1 à 3 **caractérisée en ce que** dans l'étape (ii), la durée de mise en contact entre les cellules mononucléées du sang circulant et l'éphrine-B est comprise entre 5 minutes et 6 heures.

5. Composition selon l'une quelconque des revendications 1 à 4, dans laquelle l'éphrine-B est l'éphrine-B2.

6. Composition selon l'une quelconque des revendications 1 à 5, dans laquelle la molécule de stabilisation et/ou de liaison est un fragment Fc d'une immunoglobuline.

7. Procédé de préparation d'une composition proangiogénique telle que décrite à l'une quelconque des revendications 1 à 6, comprenant les étapes suivantes :
(i) on se procure du sang périphérique comprenant des cellules mononucléées, lesdites cellules mononucléées que l'on place dans un milieu adapté, lesdites cellules mononucléées présentant des récepteurs aptes à recevoir l'éphrine-B et choisis dans le groupe consistant en EphA et EphB;
(ii) on met en contact lesdites cellules mononucléées dudit sang périphérique de l'étape (i) avec l'éphrine-B, sans soumettre lesdites cellules mononucléées aux étapes de différenciation et de maturation *ex vivo,* de manière à provoquer directement l'activation desdites cellules mononucléées.

8. Procédé selon la revendication 7, dans lequel le sang de cordon ombilical ou la moelle osseuse, ou d'un mélange des deux sont ajouté au sang périphérique comprenant les cellules mononucléées de l'étape (i).

9. Procédé selon la revendication 7 ou 8, dans lequel le milieu adapté de l'étape (i) est un plasma.

10. Procédé selon l'une quelconque des revendications 7 à 9, dans lequel l'éphrine-B est l'éphrine-B2.

11. Composition proangiogénique selon l'une quelconque des revendications 1 à 6, pour son utilisation comme médicament.

12. Composition pharmaceutique comprenant une composition proangiogénique telle que définie à l'une quelconque des revendications 1 à 6 et éventuellement un ou plusieurs excipient(s) pharmaceutiquement acceptable(s).

13. Composition proangiogénique telle que définie à l'une quelconque des revendications 1 à 6, pour son utilisation dans le traitement du diabète, de la neuropathie diabétique, de l'athérosclérose, de l'infarctus du myocarde, des accidents vasculaires cérébraux, de l'artériopathie des membres inférieurs, de l'hyperlipidémie, de l'hypercholestérolémie, de l'obésité, de l'hypertension et du vieillissement.

## Patentansprüche

1. Proangiogene Zusammensetzung, die durch ein Verfahren mit den folgenden Schritten erhalten werden kann:
(i) es wird peripheres Blut mit einkernigen Zellen beschafft, wobei die einkernigen Zellen in ein geeignetes Medium gegeben werden, wobei die einkernigen Zellen Rezeptoren aufweisen, die Ephrin-B aufnehmen können und aus der Gruppe ausgewählt sind, die aus EphA und EphB besteht;
(ii) die einkernigen Zellen des peripheren Bluts von Schritt (i) werden mit Ephrin- B in Kontakt gebracht, die mit einem Stabilisierungs- und/oder Verbindungsmolekül verbunden sind, ohne die einkernigen Zellen den Differenzierungs- und Reifungsschritten ex vivo zu unterziehen, um direkt die Aktivierung der einkernigen Zellen hervorzurufen.

2. Zusammensetzung nach Anspruch 1, in der die einkernigen Zellen aus dem peripheren Blut durch differentielle Zentrifugation isoliert werden.

3. Zusammensetzung nach Anspruch 1 oder 2, in der das Blut der Nabelschnur oder des Knochenmarks oder eines Gemischs der beiden zum peripheren Blut mit den einkernigen Zellen von Schritt (i) zugegeben wird.

4. Zusammensetzung nach einem der Ansprüche 1 bis 3, **dadurch gekennzeichnet, dass** in Schritt (ii) die Dauer des Kontakts zwischen den einkernigen Zellen des zirkulierenden Bluts und Ephrin-B zwischen 5 Minuten und 6 Stunden liegt.

5. Zusammensetzung nach einem der Ansprüche 1 bis 4, in der das Ephrin-B Ephrin-B2 ist.

6. Zusammensetzung nach einem der Ansprüche 1 bis 5, in der das Stabilisierungs-und/oder Verbindungsmolekül ein Fragment Fc eines Immunoglobulins ist.

7. Verfahren zur Herstellung einer proangiogenen Zusammensetzung nach einem der Ansprüche 1 bis 6 mit den folgenden Schritten:
(i) es wird peripheres Blut mit einkernigen Zellen beschafft, wobei die einkernigen Zellen in ein geeignetes Medium gegeben werden, wobei die einkernigen Zellen Rezeptoren aufweisen, die Ephrin-B aufnehmen können und aus der Gruppe ausgewählt sind, die aus EphA und EphB besteht;
(ii) die einkernigen Zellen des peripheren Bluts von Schritt (i) werden mit Ephrin- B in Kontakt gebracht, ohne die einkernigen Zellen den Differenzierungs- und Reifungsschritten ex vivo zu unterziehen, um direkt die Aktivierung der einkernigen Zellen hervorzurufen.

8. Verfahren nach Anspruch 7, in dem das Blut der Nabelschnur oder des Knochenmarks oder eines Gemischs der beiden zum peripheren Blut mit den einkernigen Zellen von Schritt (i) hinzugefügt wird.

9. Verfahren nach Anspruch 7 oder 8, in dem das geeignete Medium von Schritt (i) ein Plasma ist.

10. Verfahren nach einem der Ansprüche 7 bis 9, in dem das Ephrin-B Ephrin-B2 ist.

11. Proangiogene Zusammensetzung nach einem der Ansprüche 1 bis 6 für ihre Verwendung als Medikament.

12. Pharmazeutische Zusammensetzung mit einer proangiogenen Zusammensetzung nach einem der Ansprüche 1 bis 6 und eventuell einem oder mehreren pharmazeutisch verträglichen Exzipienten.

13. Proangiogene Zusammensetzung nach einem der Ansprüche 1 bis 6, für ihre Verwendung bei der Behandlung von Diabetes, diabetischer Neuropathie, Atherosklerose, Myokardinfarkt, Gehirngefäßereignissen, Arteriopathie der unteren Gliedmaßen, Hyperlipidämie, Hypercholesterinämie, Fettleibigkeit, Bluthochdruck und Alterung.

## Claims

1. A proangiogenic composition to be obtained by a method comprising the following steps of :
(i) providing peripheral blood comprising mononuclear cells, said mononuclear cells are placed in an adapted medium, said mononuclear cells having receivers being able to receive the ephrin-B and selected within the group consisting in EphA and EphB ;
(ii) contacting said mononuclear cells of said peripheral blood from step (i) with the ephrin-B associated to a stabilizing and/or a linking molecule, without subjecting said mononuclear cells to the differentiation and maturation steps *ex vivo* so as to directly cause the activation of said mononuclear cells.

2. The composition according to claim 1, wherein said mononuclear cells are separated from said peripheral blood by differential centrifugation.

3. The composition according to claim 1 or 2, wherein the blood from umbilical cord or osseous marrow, or from a mixture of both is added to the peripheral blood comprising the mononuclear cells of step (i).

4. The composition according to any of claims 1 to 3 **characterized in that** in step (ii), the contacting duration between the mononuclear cells of circulating blood and the ephrin-B is included between 5 minutes and 6 hours.

5. The composition according to any of claims 1 to 4, wherein ephrin-B is the ephrin-B2.

6. The composition according to any of claims 1 to 5, wherein the stabilizing and/or linking molecule is an immunoglobuline Fc fragment.

7. A method for preparing a proangiogenic composition according to any of claims 1 to 6, comprising the following steps:
(i) providing peripheral blood comprising mononuclear cells, said mononuclear cells are placed in an adapted medium, said mononuclear cells having receivers that can receive the ephrin-B and selected within the group consisting in EphA and EphB ;
(ii) contacting said mononuclear cells of said peripheral blood from step (i) with the ephrin-B, without subjecting said mononuclear cells to the differentiation and maturation steps *ex vivo* so as to directly cause the activation of said mononuclear cells.

8. The method according to claim 7, wherein the blood from umbilical cord or osseous marrow, or from a mixture of both is added to peripheral blood comprising the mononuclear cells of step (I).

9. The method according to claim 7 or 8, wherein the adapted medium of step (i) is a plasma.

10. The method according to any of claims 7 to 9, wherein ephrin-B is the ephrin-B2.

11. The proangiogenic composition according to any of claims 1 to 6, for its use as a drug.

12. The proangiogenic composition comprising a proangiogenic composition according to any of claims 1 to 6 and possibly one or more pharmaceutically acceptable excipients.

13. The proangiogenic composition according to any of claims 1 to 6, for its use in the treatment of diabetes, diabetic neuropathy, atherosclerosis, myocardial infarction, cerebral vascular accidents, lower limbs arteriopathy, hyperlipidemy, hypercholesterolemy, obesity, hypertension and ageing.
